# FASCICULE DE BREVET EUROPEEN

(11) **EP 1 164 922 B1**
(45) Date de publication et mention de la délivrance du brevet: **22.11.2006**
(21) Numéro de dépôt: 00900634.7
(22) Date de dépôt: 20.01.2000
(51) Int. Cl.: A61B 5/00, A61B 5/0408, A61B 5/0404

(54) **ELECTROCARDIOGRAPHE PORTABLE ET MODULE CENTRAL DE TRAITEMENT**
TRAGBARES ELEKTROKARDIOGRAMMGERÄT UND VERARBEITUNGZENTRALMODUL
PORTABLE ELECTROCARDIOGRAPH AND CENTRAL PROCESSING MODULE

(30) Priorité: 25.01.1999 FR 9900922
(43) Date de publication de la demande: 02.01.2002
(73) Titulaire: Mediag SA, 34725 Joncquières (FR)
(72) Inventeur: REBIERE, Jean-Luc, F-34725 Jonquières (FR)
(74) Mandataire: Gosse, Michel
(86) Numéro de dépôt international: PCT/FR2000/000132
(87) Numéro de publication internationale: WO 2000/042903

(56) Documents cités:
- WO-A-90/09143
- WO-A-93/19667
- WO-A-94/03106
- FR-A- 2 624 749
- FR-A- 2 636 525
- US-A- 3 732 868
- US-A- 4 583 549
- US-A- 5 465 727

## Description

L'invention est relative à un procédé et à un dispositif pour l'enregistrement des 12 dérivations cardiaques du genre comportant au moins un module portable, autonome, pourvu de moyens aptes à saisir, enregistrer et transmettre à distance, les données relatives aux enregistrements des dérivations cardiaques et un module central pourvu de moyens aptes à réceptionner et à traiter lesdites données pour effectuer notamment le tracé des enregistrements et leur archivage.

L'électrocardiographie consiste à enregistrer et analyser la régularité des phénomènes électriques qui accompagnent la contraction du coeur. Ces enregistrements sont représentés sous la forme de courbes correspondant à différents angles dénommés dérivations.
Pour effectuer un électrocardiogramme fiable, 12 dérivations standards ont été définies.
Dans l'électrocardiographe conventionnel, 10 électrodes filaires sont nécessaires pour effectuer les enregistrements correspondant aux 12 dérivations et l'appareil en question est volumineux, difficilement transportable et onéreux pour une utilisation portable.
Dans un souci de simplification, les points de saisie des données cardiaques sur le corps ont été réduits au nombre de 9 :
- 3 situés respectivement au milieu de chaque clavicule et à la hauteur de l'épine iliaque antérieure gauche;
- 6 situés dans les zones précordiales.
Grâce à cette réduction du nombre de points de saisie, des appareils plus simples ont pu voir le jour et plus particulièrement des appareils portables du genre comportant :
- uniquement des électrodes-plots (en nombre limité) placées sur le boîtier du module portable comme décrit dans le brevet US-3776228 mais nécessitant autant de positions de mesures que de dérivations à mesurer avec transfert et réinitialisation après chaque mesure et avec une limitation du nombre de dérivations mesurées;
- des électrodes-plots placées sur le boîtier du module portable et des électrodes-filaires reliées audit boîtier comme décrit dans le brevet FR-2645005 mais engendrant les mêmes inconvénients que ceux sus-mentionnés;
- uniquement des électrodes-plots (en nombre plus important) comme décrit dans le brevet US-5465727 mais selon un dispositif et un procédé totalement différents de ceux utilisés dans la demande examinée.
En outre, ces appareils ne sont pas d'un usage pratique, les mesures ne sont pas fiables de par le positionnement des électrodes.
C'est dans le but de remédier à ces divers inconvénients et d'obtenir un procédé et un appareil d'un usage simple, fiable, capable d'effectuer avec un minimum de manipulations la mesure des 12 dérivations cardiaques, que le déposant a conçu un électrocardiographe dont le procédé de mesure se caractérise essentiellement : en ce que la saisie des données relatives aux enregistrements des 12 dérivations cardiaques est effectuée à partir de 9 points corporels situés respectivement, pour les 3 premiers, au milieu de chaque clavicule et à la hauteur de l'épine iliaque antérieure gauche et, pour les 6 autres, dans les zones précordiales; en ce que ladite saisie est effectuée au moyen de seulement 3 électrodes-plots et 3 électrodes-filaires; en ce que ladite saisie s'effectue en positionnant les électrodes-filaires respectivement sur les points de saisie claviculaires et iliaques et en positionnant les électrodes-plots successivement sur les groupes de points précordiaux, pris deux à deux, autrement dit en seulement 3 positionnements du boîtier, la troisième l'électrode-plot servant d'électrode de référence; et en ce que le moyen apte à enregistrer les données relatives auxdits enregistrements, mémorise l'ensemble des données relatives aux 12 dérivations avant leur transfert vers le module central, autrement dit en une seule opération de transmission.
Quant au dispositif selon l'invention, il se caractérise essentiellement en ce qu'il comporte un boîtier, portable, pourvu de 3 électrodes plots positionnées directement sur celui-ci en des points bien spécifiques et de 3 électrodes filaires reliées audit boîtier par un câble de liaison.
Les tracés obtenus avec un tel ensemble sont identiques à ceux obtenus avec les électrocardiographes conventionnels.
Le patient peut utiliser lui-même le module portable car il est simple d'utilisation et ne nécessite aucune connaissance particulière.
Il peut ainsi obtenir un diagnostic et des conseils thérapeutiques immédiats.
La transmission des données peut se faire vers un praticien muni d'un module central n'importe où dans le monde.
L'utilisation en cabinet est également simplifiée avec transcription immédiate sur papier ordinaire et sous une présentation personnalisée.
Ce type d'appareil a pour principaux avantages :
- d'amener les connaissances du praticien auprès des malades;
- de démarrer très tôt les traitements spécifiques;
- d'effectuer de la télémédecine avec diagnostic et surveillance à distance des malades;
- d'améliorer la recherche cardiologique par la constitution de bases de données facilement connectables et exportables.

Les caractéristiques et les avantages de l'invention vont apparaître plus clairement à la lecture de la description détaillée qui suit d'au moins un mode de réalisation préféré de celle-ci donné à titre d'exemple non limitatif et représenté aux dessins annexés.
Sur ces dessins :
- la figure 1 est une vue indiquant la position sur le corps des 9 points de mesure permettant la saisie des données relatives aux 12 dérivations cardiaques;
- la figure 2 est une vue en coupe longitudinale AA du boîtier,
- la figure 3 est une vue de dessus dudit boîtier.

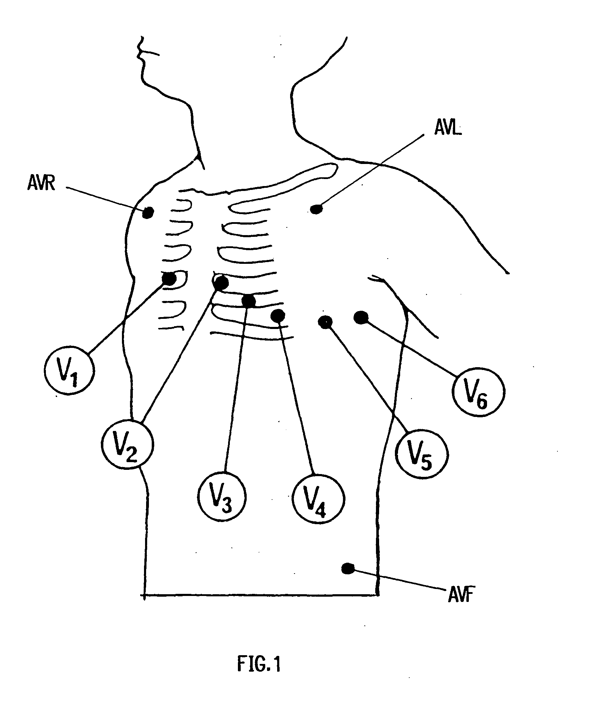

Les 9 points corporels de saisie des données relatives aux enregistrements des 12 dérivations cardiaques, sont situés (fig.1) respectivement :
- pour les 3 premiers, au milieu de chaque clavicule (AVR) et (AVL) et à la hauteur de l'épine iliaque antérieure gauche (AVF);
- pour les 6 autres (V1) à (V6), dans les zones précordiales.

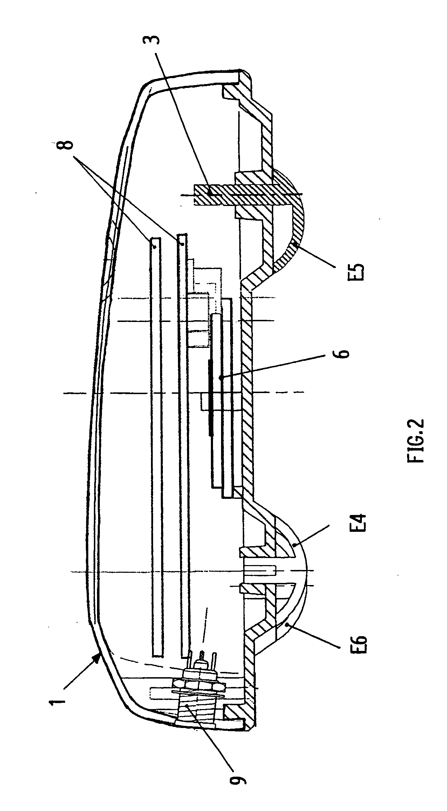

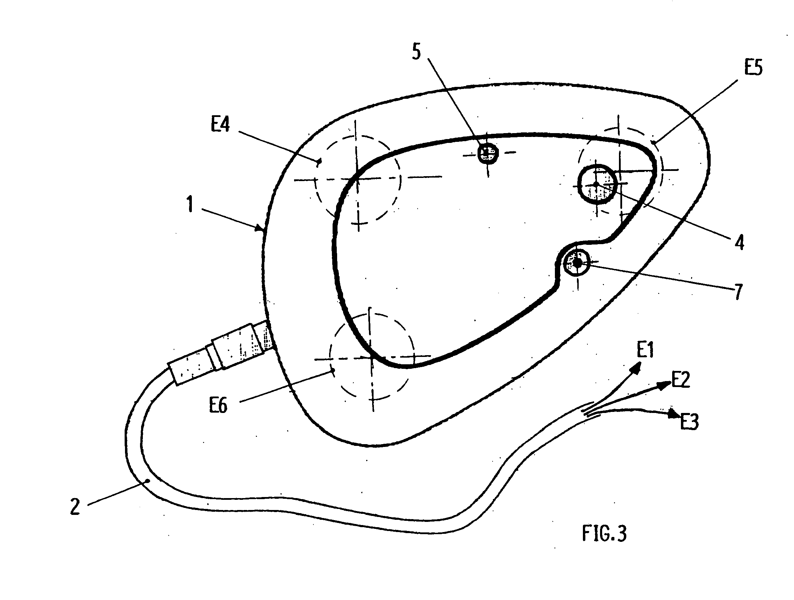

Le dispositif représenté aux figures 2 et 3 est du genre comportant au moins un module portable autonome, pourvu de moyens aptes à saisir, enregistrer et transmettre à distance les données relatives aux enregistrements des dérivations cardiaques et un module central (non représenté) pourvu de moyens aptes à réceptionner et à traiter lesdites données pour effectuer notamment le tracé des enregistrements correspondants.
Ledit dispositif comporte essentiellement un boîtier (1), portable, pourvu de 3 électrodes plots (E4, E5, E6) positionnées directement sur celui-ci en des points bien spécifiques et de 3 électrodes filaires (E1, E2, E3) reliées audit boîtier par un câble de liaison (2).
Le procédé de mesure des données relatives aux 12 dérivations cardiaques se caractérise en ce que :
a) la saisie des données relatives aux enregistrements des 12 dérivations cardiaques est effectuée à partir de 9 points corporels (AVR), (AVL) (AVF) et (V1) à (V6) tels que définis ci-avant;
b) ladite saisie est effectuée au moyen de seulement 3 électrodes-plots (E4), (E5) et (E6) et 3 électrodes-filaires (E1), (E2) et (E3);
c) ladite saisie s'effectue en positionnant les électrodes-filaires (E1), (E2), et (E3) respectivement sur les points de saisie (AVR), (AVL) et (AVF) et en positionnant les électrodes-plots (E4) et (E5) successivement sur les groupes de points, pris deux à deux, (V1) et (V2), (V3) et (V4), (V5) et (V6), autrement dit en seulement 3 positionnements du boîtier, l'électrode-plot (E6) servant d'électrode de référence;
d) le moyen apte à enregistrer les données relatives auxdits enregistrements, mémorise l'ensemble des données relatives aux 12 dérivations avant leur transfert vers le module central, autrement dit en une seule opération de transmission.
Selon diverses particularités de réalisation du dispositif :
- le moyen de transmission du module portable comporte un module de compression du signal numérique transmis et le moyen de réception du module central comporte un module de décompression du signal numérique reçu;
- l'une (E5) des électrodes-plots (E4,E5) est montée mobile sur le boîtier de manière à faire varier la distance existant entre les deux-dites électrodes en fonction de la morphologie thoracique du patient;
- le déplacement de l'électrode mobile (E5) est réalisé par sa mise en rotation autour de son axe de fixation (3) au boîtier qui est monté excentré sur celle-ci;
- le module central comporte une interface de réception, pourvue des moyens adaptés pour réceptionner et décompresser le signal numérique reçu, relié à l'entrée série d'un moyen de traitement du type "PC" adapté pour visualiser notamment les résultats des enregistrements cardiaques;
- le module portable comporte un bouton de commande unique (4) adapté pour d'une part lancer, en une seule pression, les 3 cycles d'enregistrements correspondant aux 3 positionnements du boîtier sur les zones précordiales et d'autre part, en une seule nouvelle pression, le transfert des données enregistrées vers le module central;
- le bouton de commande (4) est associé à un voyant bicolore (5) qui est adapté pour indiquer, dans un état de couleur, la période de stabilisation des signaux, et, dans l'autre état de couleur, la période d'acquisition des données de mesure et ce, pour une position bien spécifique du boîtier;
- le bouton de commande (4) et le voyant bicolore (5) sont associés à un avertisseur sonore qui est adapté pour indiquer le début et la fin de chacune des 3 périodes de mesures et l'ordre chronologique desdites périodes donc des 3 positions du boîtier sur les zones précordiales.Le boîtier comporte sa propre alimentation, par piles par exemple.
Il peut comporter un moyen de suspension au cou du patient.
Les fils peuvent se rétracter dans celui-ci.
Le module central peut comporter deux bornes permettant son branchement entre la base et le combiné.

Bien entendu, l'invention n'est pas limitée aux modes de réalisation décrits et représentés pour lesquels on pourra prévoir d'autres variantes en particulier dans les formes et la nature des matériaux utilisés, les positions et les types d'électrodes-plots utilisées, les modules de compression et de décompression du signal numérique, les types de boutons, de voyants et autres sous-ensembles.

## Revendications

1. Procédé de mesure des données relatives aux 12 dérivations cardiaques utilisant au moins un module portable, autonome, pourvu de moyens aptes à saisir consistant en des électrodes plots positionnées sur le boîtier dudit module et en des électrodes filaires reliées à ce dernier, à enregistrer et à transmettre à distance, lesdites données et un module central pourvu de moyens aptes à réceptionner et à traiter lesdites données pour effectuer notamment le tracé des enregistrements correspondants;
**caractérisé en ce que** la saisie des données relatives aux enregistrements des 12 dérivations cardiaques est effectuée à partir de 9 points corporels situés respectivement, pour les 3 premiers, au milieu de chaque clavicule (AVR) et (AVL) et à la hauteur de l'épine iliaque antérieure gauche (AVF) et, pour les 6 autres (V1) à (V6), dans les zones précordiales;
**en ce que** ladite saisie est effectuée au moyen de seulement 3 électrodes-plots (E4), (E5) et (E6) et 3 électrodes-filaires (E1), (E2) et (E3);
**en ce que** ladite saisie s'effectue en positionnant les électrodes-filaires (E1), (E2), et (E3) respectivement sur les points de saisie (AVR), (AVL) et (AVF) et en positionnant les électrodes-plots (E4) et (E5) successivement sur les groupes de points, pris deux à deux, (V1) et (V2), (V3) et (V4), (V5) et (V6), autrement dit en seulement 3 positionnements du boîtier, l'électrode-plot (E6) servant d'électrode de référence;
et **en ce que** le moyen apte à enregistrer les données relatives auxdits enregistrements, mémorise l'ensemble des données relatives aux 12 dérivations avant leur transfert vers le module central, autrement dit en une seule opération de transmission.

2. Dispositif pour la mesure des données relatives aux 12 dérivations cardiaques comportant au moins un module portable, autonome, pourvu de moyens aptes à saisir consistant en des électrodes plots positionnées sur le boîtier dudit module et en des électrodes filaires reliées à ce dernier, à enregistrer et à transmettre à distance, lesdites données et un module central pourvu de moyens aptes à réceptionner et à traiter lesdites données pour effectuer notamment le tracé des enregistrements correspondants ;
**caractérisé en ce que** le module portable comporte 3 électrodes filaires (E1, E2, E3) et 3 électrodes plots (E4,E5,E6) dont l'une (E5) est montée mobile sur le boîtier de manière à faire varier la distance qui la sépare de l'une (E4) des deux autres électrodes au moyen de sa mise en rotation autour de son axe de fixation (3) au boîtier qui est monté excentré sur celle-ci.

3. Dispositif, selon la revendication 2, **caractérisé en ce que** le module portable comporte un bouton de commande unique (4) adapté pour d'une part lancer, en une seule pression, les 3 cycles d'enregistrements correspondant aux 3 positionnements du boîtier sur les zones précordiales et d'autre part, en une seule nouvelle pression, le transfert des données enregistrées vers le module central.

4. Dispositif, selon la revendication 3, **caractérisé en ce que** le bouton de commande (4) est associé à un voyant bicolore (5) qui est adapté pour indiquer, dans un état de couleur donné, la période de stabilisation des signaux, et, dans l'autre état de couleur, la période d'acquisition des données de mesure et ce, pour une position bien spécifique du boîtier.

5. Dispositif, selon la revendication 4, **caractérisé en ce que** le bouton de commande (4) et le voyant bicolore (5) sont associés à un avertisseur sonore qui est adapté pour indiquer le début et la fin de chacune des 3 périodes de mesures et l'ordre chronologique desdites périodes donc des 3 positions du boîtier sur les zones précordiales.

## Claims

1. Method to measure data relating to the 12 cardiac derivations using at least one standalone portable module, equipped with means suitable for inputting consisting of block electrodes positioned on the housing of said module and of wired electrodes connected thereto, saving and transmitting said data remotely and a central module equipped with means suitable for receiving and processing said data particularly to plot the corresponding records;
**characterised in that** the input of the data relating to the records of the 12 cardiac derivations is performed from 9 body points located respectively, in the case of the first three, in the centre of each collarbone (AVR) and (AVL) and at the level of the left anterior iliac spine (AVF) and, in the case of the six others (V1) to (V6), in the precordial zones;
**in that** said input is performed by means of only three block electrodes (E4), (E5) et (E6) and three wired electrodes (E1), (E2) and (E3);
**in that** said input is performed by positioning the wired electrodes (E1), (E2), and (E3) on the input points (AVR), (AVL) and (AVF) respectively and by positioning the block electrodes (E4) and (E5) successively on the groups of points, taken in pairs, (V1) and (V2), (V3) and (V4), (V5) and (V6), in other words, only using three positions, the block electrode (E6) serving as a reference electrode;
and **in that** the means suitable for saving the data relating to said records, store all the data relating to the 12 bypasses before the transfer thereof to the central modules, in other words, in a single transmission operation.

2. Device to measure the data relating to the 12 cardiac derivations comprising at least one standalone portable module, equipped with means suitable for inputting consisting of block electrodes positioned on the housing of said module and of wired electrodes connected thereto, saving and transmitting said data remotely, and a central module equipped with means suitable for receiving and processing said data particularly to plot the corresponding records;
**characterised in that** the portable module comprises three wired electrodes (E1, E2, E3) and three block electrodes (E4,E5,E6) wherein one (E5) is mounted mobile on the housing so as to vary the distance separating it from one (E4) of the two other electrodes by means of the rotation thereof around its attachment axis (3) to the housing which is mounted offset thereon.

3. Device, according to claim 2, **characterised in that** the portable module comprises a single control knob (4) suitable for, firstly, starting, with a single press, the three recording cycles corresponding to the three housing positions on the precordial zones and, secondly, the transfer of the saved data to the central module.

4. Device, according to claim 3, **characterised in that** the control knob (4) is associated with a two-coloured indicator light (5) which is suitable for indicating, in a given colour status, the signal stabilisation period and, in the other colour status, the measurement data acquisition period, for a specific housing position.

5. Device, according to claim 4, **characterised in that** control knob (4) and the two-coloured indicator light (5) are associated with an audio warning device which is suitable for indicating the start and the end of each of the three measurement periods and the chronological order of said periods and therefore the three positions of the housing on the precordial zones.

## Patentansprüche

1. Verfahren zur Messung der Daten von 12 Herzableitungen, mindestens ein tragbares, unabhängiges Modul verwendend, ausgestattet mit Mitteln zur Erfassung, bestehend aus auf dem Gehäuse des besagten Moduls positionierten Kontaktelektroden und aus mit diesem verbundenen drahtgebundenen Elektroden, zur Aufzeichnung und zur Fernübertragung der besagten Daten, und ein Zentralmodul, ausgestattet mit Mittel zum Empfang und zur Verarbeitung der besagten Daten, um vor allem die grafische Darstellung der entsprechenden Aufzeichnungen durchzuführen;
**dadurch gekennzeichnet, dass** die Erfassung der Daten bezüglich der Aufzeichnungen der 12 Herzabteilungen ausgehend von 9 Körperpunkten durchgeführt wird, die sich jeweils im Hinblick auf die 3 ersten Punkte in der Mitte jeder Clavicula (AVR) und (AVL) und in Höhe der Spina iliaca anterior links (AVF) und für die 6 anderen (V1) bis (V6) in den präkordialen Bereichen befinden;
dadurch, dass die besagte Erfassung mit nur 3 Kontaktelektroden (E4), (E5) und (E6) und 3 drahtgebundenen Elektroden (E1), (E2) und (E3) durchgeführt wird;
dadurch, dass die besagte Erfassung durchgeführt wird, indem die drahtgebundenen Elektroden (E1), (E2) und (E3) jeweils auf den Erfassungspunkten (AVR), (AVL) und (AVF) positioniert werden und indem die Kontaktelektroden (E4) und (E5) nacheinander jeweils paarweise auf den Punktgruppen (V1) und (V2), (V3) und (V4), (V5) und (V6) positioniert werden, anders ausgedrückt, mit nur 3 Positionierungen des Gehäuses, wobei die Kontaktelektrode (E6) als Bezugselektrode dient;
und dadurch, dass das Mittel zur Aufzeichnung der den besagten Erfassungen entsprechenden Daten alle Daten der 12 Ableitungen vor ihrer Weiterleitung an das Zentralmodul speichert, anders gesagt, in einem einzigen Übertragungsvorgang.

2. Vorrichtung zur Messung der Daten von 12 Herzableitungen, mindestens ein tragbares, unabhängiges Modul umfassend, ausgestattet mit Mitteln zur Erfassung, bestehend aus auf dem Gehäuse des besagten Moduls positionierten Kontaktelektroden und aus mit diesem verbundenen drahtgebundenen Elektroden, zur Aufzeichnung und zur Fernübertragung der besagten Daten, und ein Zentralmodul, ausgestattet mit Mittel zum Empfang und zur Verarbeitung der besagten Daten, um vor allem die grafische Darstellung der entsprechenden Aufzeichnungen durchzuführen;
**dadurch gekennzeichnet, dass** das tragbare Modul 3 drahtgebundene Elektroden (E1, E2, E3) und drei Kontaktelektroden (E4, E5, E6) umfasst, von denen eine (E5) beweglich auf dem Gehäuse angebracht ist, um den Abstand, der sie von einer (E4) der anderen zwei Elektroden trennt, durch Drehen um ihre Befestigungsachse (3) am Gehäuse, die außermittig an dieser befestigt ist, verändern zu können.

3. Vorrichtung nach Anspruch 2, **dadurch gekennzeichnet, dass** das tragbare Modul einen einzigen Bedienungsknopf (4) umfasst, mit dem einerseits durch einmaliges Drücken die den 3 Positionen des Gehäuses auf den präkordialen Bereichen entsprechenden 3 Aufzeichnungszyklen gestartet werden können, und andererseits durch erneutes einmaligen Drücken die Übertragung der aufgezeichneten Daten zum Zentralmodul.

4. Vorrichtung nach Anspruch 3, **dadurch gekennzeichnet, dass** der Bedienungsknopf (4) mit einer zweifarbigen Anzeigelampe (5) verbunden ist, die mit einer bestimmten Farbe die Periode der Signalstabilisierung anzeigt und mit der anderen Farbe die Periode der Erfassung der Messdaten, und zwar für eine bestimmte Gehäuseposition.

5. Vorrichtung nach Anspruch 4, **dadurch gekennzeichnet, dass** der Bedienungsknopf (4) und die zweifarbige Anzeigelampe (5) mit einem akustischen Signalgeber verbunden sind, der den Beginn und das Ende jeder der 3 Messperioden und die chronologische Reihenfolge der besagten Perioden anzeigt, das heißt der 3 Gehäusepositionen auf den präkordialen Bereichen.
